# EUROPEAN PATENT APPLICATION

(11) **EP 4 647 499 A1**
(43) Date of publication of application: **12.11.2025**
(21) Application number: 24757179.7
(22) Date of filing: 13.02.2024
(51) Int. Cl.: C12P 7/54, C12P 13/12

(54) **METHOD FOR RECOVERING ACETIC ACID FROM FERMENTATION BROTH OR FERMENTATION WASTE LIQUOR AND REUSING SAME**

(30) Priority: 13.02.2023 KR 20230018840
(71) Applicant: CJ CheilJedang Corporation, Seoul 04560 (KR)
(72) Inventor: PARK, Woohyung, Seoul 04560 (KR); HONG, Soon Won, Seoul 04560 (KR); HWANG, In Seok, Seoul 04560 (KR); HONG, Seok Bin, Seoul 04560 (KR); KIM, Jun-Woo, Seoul 04560 (KR)
(74) Representative: Meissner Bolte Partnerschaft mbB
(86) International application number: PCT/KR2024/002012
(87) International publication number: WO 2024/172452

(57) **Abstract**

Provided are a method of recovering acetic acid in the form of an acetic acid solution from a fermentation broth or a waste liquor thereof, and/or a method of reusing the recovered acetic acid in a fermentation process.

## Description

### [Technical Field]

The present disclosure relates to a method of recovering acetic acid in the form of an acetic acid solution from a fermentation broth or a waste liquor thereof, and/or a method of reusing the recovered acetic acid in a fermentation process.

### [Background Art]

In some cases, the production of a target substance through fermentation produces acetic acid. For example, US 9029105 B2 discloses a method of producing L-methionine from O-acetyl-L-homoserine via a converting enzyme, from which it can be seen that acetic acid is produced as a by-product. However, when such acetic acid is handled as wastewater, it must be diluted to at least 10 wt% or less and then neutralized before being drained, which causes a problem in that energy and cost are required for wastewater treatment. Accordingly, a method of recovering residual acetic acid from a fermentation broth comprising a large amount of acetic acid or a waste liquor thereof and reusing the same is contemplated.

Meanwhile, US 9752167 B2 mentions a method of recovering acetic acid from a fermentation broth or waste liquor thereof and a process of reusing the recovered acetic acid in fermentation. However, since acetic acid is recovered in a form dissolved in an organic acid, a separate process of separating acetic acid by an organic solvent extraction process is required. The organic solvent extraction process is complicated because it incurs costs due to the use of solvent and requires an additional solvent recovery process, which reduces economic feasibility. In addition, even a trace amount of the available organic solvent may act as a toxin in the fermentation process, making it difficult to apply to the fermentation process.

### [Disclosure]

### [Technical Problem]

An object of the present disclosure is to provide a method of recovering acetic acid in the form of an acetic acid solution from a fermentation broth or fermentation waste liquor comprising acetic acid.

Another object of the present disclosure is to provide a method of reusing acetic acid by separating the acetic acid in the form of an acetic acid solution from a fermentation broth or fermentation waste liquor comprising acetic acid.

### [Technical Solution]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

To achieve the above objects, an aspect of the present disclosure provides a method of recovering acetic acid from a fermentation broth or fermentation waste liquor, the method comprising the steps of:
(a) adding a basic substance to the fermentation broth or fermentation waste liquor comprising acetic acid to adjust pH to 4.6 to 6.0; and
(b) concentrating the pH-adjusted fermentation broth or fermentation waste liquor by a reverse osmosis process to obtain an acetic acid solution.

Unlike the existing method of recovering acetic acid in the form dissolved in an organic acid (US 9752167 B2), the method of recovering acetic acid from a fermentation broth in the form of an acetic acid solution and reusing the same of the present disclosure may recover acetic acid in the form of an acetic acid solution without a separate extraction process, thereby recovering acetic acid with a high yield in the form of an acetic acid solution from a fermentation broth or fermentation waste liquor without using an organic solvent. Accordingly, the method is advantageous in that the process is simplified, thereby increasing economic efficiency, and no organic solvent remains, and thus it may be applied to a fermentation process, and the obtained acetic acid may be used immediately without a re-dissolution process. Therefore, when the acetic acid recovered according to the method of the present disclosure is reused in a fermentation process with a long fermentation time, for example, in a fermentation process of producing amino acids such as L-methionine, etc., there are advantages of increasing the productivity and decreasing the culture time, and thus the method may be very usefully applied.

As used herein, the term "fermentation broth" may comprehensively refer to a medium comprising acetic acid, which is obtained by culturing a microorganism producing a fermentation product, a culture comprising the microorganism cultured together with the medium, or an enzyme conversion solution thereof, etc.

Specifically, the fermentation broth may comprise (1) a fermentation broth of a target substance, a precursor thereof, or a derivative thereof, comprising acetic acid, (2) a fermentation broth comprising a target substance or a derivative thereof produced by a strain producing a precursor of the target substance, a substrate, and a converting enzyme; and acetic acid, or (3) a fermentation broth comprising a target substance or a derivative thereof produced by adding a converting enzyme or a microorganism expressing the converting enzyme; and a substrate to a precursor fermentation broth of the target substance; and acetic acid, but is not limited thereto.

More specifically, the fermentation broth may comprise an amino acid or a derivative thereof prepared by adding (i) O-acylhomoserine; and (ii) O-acylhomoserine converting enzyme or a microorganism expressing the same, but is not limited thereto.

As used herein, the term "O-acylhomoserine" comprises O-acetyl-L-homoserine, O-succinyl-L-homoserine, propionyl homoserine, acetoacetyl homoserine, coumaroyl homoserine, malonyl homoserine, hydroxymethylglutaryl homoserine, pimelyl homoserine, but is not limited thereto.

More specifically, the fermentation broth may be a fermentation broth comprising an amino acid or a derivative thereof prepared by adding a converting enzyme or a microorganism expressing the same to an O-acetyl-L-homoserine fermentation broth; and acetic acid. In addition, the fermentation broth may be specifically a fermentation broth comprising L-homocysteine, L-methionine, or a derivative thereof prepared by adding O-acetylhomoserine sulfhydrylase or a microorganism expressing the same; and methyl mercaptan (CH₃SH) to an O-acetyl-L-homoserine fermentation broth; and acetic acid (US 8426171 B2).

The type of the "fermentation product" is not limited as long as the fermentation broth comprises acetic acid. However, it may be specifically a target substance produced by fermentation, a derivative or precursor thereof. In addition, the target substance may be an amino acid, for example, L-homocysteine or L-methionine, but is not limited thereto. In addition, the derivative of the target substance may be a derivative of an amino acid, specifically, a derivative of L-homocysteine or a derivative of L-methionine. In addition, the precursor of the target substance may be a precursor of an amino acid, specifically, a precursor of L-homocysteine or a precursor of L-methionine. More specifically, it may be O-acetyl-L-homoserine, but is not limited thereto.

As used herein, the term "fermentation waste liquor" may be a liquid obtained by separating and removing part or all of the fermentation product from the fermentation broth, but is not limited thereto. Specifically, the fermentation waste liquor may be a liquid obtained by separating and removing part or all of an amino acid, a derivative or precursor thereof from a fermentation broth comprising the amino acid, the derivative or precursor thereof, and acetic acid, but is not limited thereto. For example, the fermentation waste liquor may be a liquid obtained by separating and removing part or all of an amino acid or a derivative thereof from a fermentation broth comprising the amino acid, the derivative thereof, and acetic acid, or a liquid obtained by separating and removing part or all of an amino acid or a derivative thereof from a fermentation broth comprising the amino acid or the derivative thereof, which is produced by a substrate and a converting enzyme or a microorganism producing the same in an amino acid precursor fermentation broth, and acetic acid. More specifically, the fermentation waste liquor may be, for example, a liquid obtained by separating and removing part or all of L-homocysteine, L-methionine, or a derivative thereof from a fermentation broth comprising L-homocysteine, L-methionine, or the derivative thereof, which is produced by adding O-acetylhomoserine sulfhydrylase or a microorganism expressing the same; and methyl mercaptan (CH₃SH) to an O-acetyl-L-homoserine fermentation broth; and acetic acid, but is not limited thereto.

In the present disclosure, the step (a) (pH adjustment step) is a step of adjusting the pH of the fermentation broth or fermentation waste liquor comprising acetic acid. The pH of the fermentation broth or fermentation waste liquor comprising acetic acid, which is obtained in the pH adjustment step may be adjusted to 4.6 to 6.0, specifically, 4.8 to 6.0, and more specifically, 5.0 to 6.0.

When the pH of the fermentation broth or fermentation waste liquor comprising acetic acid, which is obtained in the pH adjustment step is lower than 4.6, there is a problem in that the final acetic acid recovery rate is less than 50%.

Meanwhile, the reverse osmosis process is based on a principle of separating, from a solution, a solvent that passes through a semipermeable membrane and a solute that does not pass through the semipermeable membrane by applying a pressure greater than the osmotic pressure. In this regard, ammonium acetate generated in the neutralization reaction of ammonia and acetic acid cannot pass through the semipermeable membrane and is thus separated. However, when ammonium acetate is excessively produced during this process, a problem occurs where the semipermeable membrane is blocked and the solvent is not separated.

In other words, when the pH of the fermentation broth or fermentation waste liquor comprising acetic acid, which is obtained in the pH adjustment step, exceeds 6.0, there is a problem in that the reverse osmosis process will experience equipment limitations. In addition, the pH adjustment step in the present disclosure may be performed by adding a basic substance to the fermentation broth or fermentation waste liquor comprising acetic acid.

In one specific embodiment, the basic substance may be ammonia or sodium hydroxide, but is not limited thereto. Specifically, the pH adjustment step may be performed by adding ammonia gas (NH₃(g)) or ammonia water (NH₄OH(liq)).

In one specific embodiment, the method may further comprise, before the step (a), (i) a step of preparing a fermentation broth comprising L-homocysteine, L-methionine or a derivative thereof by using microorganisms in a fermentation medium comprising O-acylhomoserine, and (ii) a step of obtaining the fermentation broth or fermentation waste liquor comprising acetic acid of the step (a) from the fermentation broth; or the fermentation waste liquor obtained by removing L-homocysteine, L-methionine, or the derivative thereof from the fermentation broth, but is not limited thereto.

In one specific embodiment, the step (ii) may further comprise a step of evaporating and cooling the fermentation broth; or the fermentation waste liquor obtained by removing L-homocysteine, L-methionine, or the derivative thereof from the fermentation broth to obtain condensate water comprising water and acetic acid, but is not limited thereto.

In one specific embodiment, the condensate water may comprise acetic acid at a concentration of 5 g/L to 15 g/L, but is not limited thereto.

In the present disclosure, the step (b) is a step of concentrating the pH-adjusted fermentation broth or fermentation waste liquor by a reverse osmosis process to obtain an acetic acid solution.

As used herein, the term "concentrating" is not limited to a method, as long as it is able to increase the concentration of acetic acid ions comprised in the fermentation broth or fermentation waste liquor, and may be performed by a method known in the art. Specifically, the concentrating may be performed by evaporating, heating, depressurizing, ventilating, freezing, etc., but is not limited thereto.

In one specific example, the step (b) may comprise a step of re-concentrating the obtained acetic acid solution, but is not limited thereto.

By performing the reverse osmosis process, the acetic acid recovery rate may be increased, and a retentate from which various suspended substances have been removed by filtering may be obtained.

As used herein, the term "retentate" may refer to a solution with an increased solute concentration as a result of the reverse osmosis process.

In any one embodiment of the above-described embodiments, the obtained acetic acid solution may comprise acetic acid at a concentration of 25 g/L to 45 g/L, but is not limited thereto.

In any one embodiment of the above-described embodiments, the re-concentrated solution prepared in the re-concentration step may comprise acetic acid at a concentration of 200 g/L to 400 g/L, but is not limited thereto.

The method of recovering acetic acid in the form of an acetic acid solution from the fermentation broth and reusing the same of the present disclosure may recover acetic acid with a high yield in the form of an acetic acid solution from the fermentation broth or fermentation waste liquor without using an organic solvent through a simple process consisting only of pH adjustment and concentration of the fermentation broth or fermentation waste liquor comprising acetic acid without a separate extraction process. Accordingly, the method is advantageous in that the process is simplified, thereby increasing economic efficiency, and no organic solvent remains, and thus it may be applied to a fermentation process, and the obtained acetic acid in the form of an acetic acid solution may be used immediately without a re-dissolution process.

In one specific embodiment, the acetic acid recovered by the method of the present disclosure may comprise acetic acid and/or a salt of acetic acid and/or an ester of acetic acid.

In one specific embodiment, the acetic acid solution recovered by the method of the present disclosure may be, but is not limited to, an aqueous acetic acid solution.

Another aspect of the present disclosure provides a method of reusing acetic acid from a fermentation broth or fermentation waste liquor, the method comprising the steps of:
(a) adding a basic substance to a fermentation broth or fermentation waste liquor comprising acetic acid to adjust pH to 4.6 to 6.0;
(b) concentrating the pH-adjusted fermentation broth or fermentation waste liquor by a reverse osmosis process to obtain an acetic acid solution; and
(c) introducing the acetic acid solution to a medium.

In the present disclosure, the step (c) is a step of reusing the acetic acid solution obtained in the step (b) in the fermentation process.

In the present disclosure, the reusing step is a step of using the acetic acid in the form of the acetic acid solution, which is recovered by the method of the present disclosure, as an acetic acid and/or carbon source in various fermentation media that require the acetic acid and/or carbon source. The fermentation media of the present disclosure are not limited as long as they are media used in fermentation requiring the acetic acid and/or carbon source with respect to the objects of the present disclosure.

Still another aspect of the present disclosure provides a method of recovering acetic acid from a fermentation broth or fermentation waste liquor, the method comprising the steps of:
(a') producing O-acetyl-L-homoserine by a fermentation process using a microorganism producing O-acetyl-L-homoserine;
(b') reacting O-acetyl-L-homoserine produced in the step (a') with methyl mercaptan (CH₃SH) in the presence of O-acetylhomoserine sulfhydrylase or a microorganism expressing the same to produce a fermentation broth of L-homocysteine, L-methionine, or a derivative thereof;
(c') obtaining the fermentation broth; or the fermentation waste liquor obtained by removing L-homocysteine, L-methionine, or the derivative thereof from the fermentation broth;
(d') adding a basic substance to the fermentation broth of the step (a') or the fermentation waste liquor of the step (b') to adjust pH to 4.6 to 6.0; and
(e') concentrating the pH-adjusted fermentation broth or fermentation waste liquor by a reverse osmosis process to obtain an acetic acid solution.

Still another aspect of the present disclosure provides a method of reusing acetic acid from a fermentation broth or fermentation waste liquor, the method comprising the steps of:
(a') producing O-acetyl-L-homoserine by a fermentation process using a microorganism producing O-acetyl-L-homoserine;
(b') producing a fermentation broth of L-homocysteine, L-methionine, or a derivative thereof by reacting O-acetyl-L-homoserine produced in the step (a') with methyl mercaptan (CH₃SH) in the presence of O-acetylhomoserine sulfhydrylase or a microorganism expressing the same;
(c') obtaining the fermentation broth; or the fermentation waste liquor obtained by removing L-homocysteine, L-methionine, or the derivative thereof from the fermentation broth;
(d') adding a basic substance to the fermentation broth of the step (a') or the fermentation waste liquor of the step (b') to adjust pH to 4.6 to 6.0;
(e') concentrating the pH-adjusted fermentation broth or fermentation waste liquor by a reverse osmosis process to obtain an acetic acid solution; and
(f') introducing the acetic acid solution to a medium.

In the present disclosure, the step (a') is a step of producing O-acetyl-L-homoserine through a fermentation process using a microorganism producing O-acetyl-L-homoserine. The microorganism producing O-acetyl-L-homoserine may be any known microorganism capable of producing O-acetyl-L-homoserine, and as a nonlimiting example, a microorganism with enhanced O-acetyl-L-homoserine production ability (US 9029105 B2) may be used.

In the present disclosure, the step (b') is a step of converting O-acetyl-L-homoserine into L-homocysteine, L-methionine, or a derivative thereof by reacting with methyl mercaptan (CH₃SH) under the catalytic action of O-acetylhomoserine sulfhydrylase enzyme.

In addition, in the present disclosure, the conversion reaction may be performed using not only the O-acetylhomoserine sulfhydrylase enzyme but also a microorganism expressing the enzyme. The enzyme and the microorganism expressing the enzyme may be obtained through means and methods known to those skilled in the art. Specifically, as the O-acetylhomoserine sulfhydrylase enzyme, an enzyme described in US 9029105 B2, etc. may be used, but is not limited thereto.

In addition, in the present disclosure, the methyl mercaptan (CH₃SH) may be added in various forms.

In the present disclosure, a step of evaporating and cooling the fermentation broth of the step (b') or the fermentation waste liquor of the step (c') to obtain condensate water comprising water and acetic acid may be further comprised between the steps (c') and (d'), but is not limited thereto.

In one specific embodiment, the condensate water may comprise acetic acid at a concentration of 5 g/L to 15 g/L, but is not limited thereto.

In the present disclosure, the steps (d') to (f') are the same as the method of recovering and reusing acetic acid in the form of an acetic acid solution from the fermentation medium as described in the present disclosure.

One embodiment of the method of the present disclosure may further comprise a step of reusing the acetic acid solution of the step (f') as an acetic acid and/or carbon source in a fermentation medium.

In the present disclosure, the reusing step is a step of using acetic acid in the form of an acetic acid solution recovered by the method of the present disclosure as an acetic acid and/or carbon source in various fermentation media requiring the acetic acid and/or carbon source. With respect to the objects of the present disclosure, the fermentation medium of the present disclosure is not limited as long as it is a medium used in fermentation requiring the acetic acid and/or carbon source.

### [Advantageous Effects]

Unlike the existing method of recovering acetic acid in the form dissolved in an organic acid (US 9260732 B2), the present disclosure investigated for the first time a method of recovering acetic acid in the form of an acetic acid solution without a separate extraction process, thereby recovering acetic acid in the form of an acetic acid solution from a fermentation broth or fermentation waste liquor without using an organic solvent, and reusing the same in a fermentation process.

The method of recovering acetic acid in the form of an acetic acid solution from a fermentation broth and reusing the same of the present disclosure may recover acetic acid in the form of an acetic acid solution from a fermentation broth or fermentation waste liquor without using an organic solvent and may reuse the same in the fermentation process, and therefore, there are no problems with organic solvents, such as the possibility of acting as a toxin in the fermentation process, additional costs incurred due to the use of solvents, and the complexity of the process due to the addition of a solvent recovery process, etc. In addition, since the obtained acetic acid may be used immediately without a re-dissolution process, it may be very usefully applied to the fermentation process.

### [Brief Description of the Drawing]

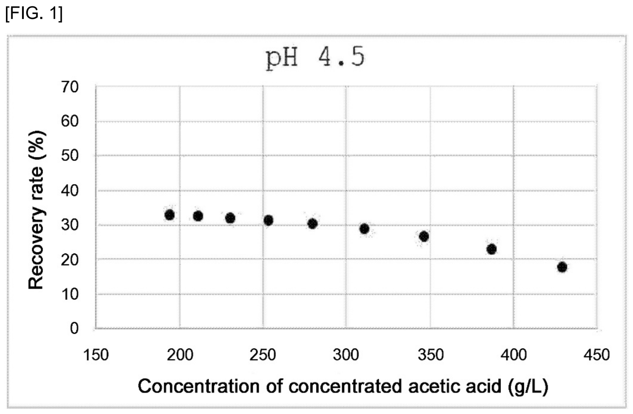

FIG. 1 is a graph showing the results of examining a recovery rate of acetic acid according to the acetic acid concentration of a re-concentrated solution when the pH of condensate water comprising acetic acid is less than 4.6.

### [Detailed Description of Preferred Embodiments]

Hereinafter, the present disclosure will be described in detail with reference to the following exemplary embodiments. However, the following exemplary embodiments are only for illustrating the present disclosure, and the scope of the present disclosure is not intended to be limited by the following exemplary embodiments.

### Example 1. Preparation of Fermentation Broth or Fermentation Waste Liquor Comprising Acetic Acid (Condensate Water Comprising Acetic Acid)

A microorganism producing O-acetyl-L-homoserine was cultured to obtain an O-acetyl-L-homoserine fermentation broth, and then the fermentation broth was reacted with methyl mercaptan (CH₃SH) using O-acetylhomoserine sulfhydrylase to obtain a fermentation broth comprising L-homocysteine, L-methionine, or a derivative thereof (US 9029105 B2). Subsequently, a fermentation waste liquor obtained by separating and removing part or all of L-homocysteine, L-methionine, or the derivative thereof from the fermentation broth was evaporated and cooled to obtain condensate water comprising water and acetic acid.

In detail, in the process of producing L-methionine using O-acetyl-L-homoserine produced through fermentation as a precursor for the enzymatic reaction, three types of crystallization tubes (crystallization tube A, crystallization tube B, crystallization tube C) and two types of concentration tubes (concentration tube A, concentration tube B) were used. The condensate water generated from crystallization tube A was 882.5 kl and comprised acetic acid at a concentration of 7.2 g/L. The condensate water generated from crystallization tube B was 1105 kl and comprised acetic acid at a concentration of 9.6 g/L. They were mixed in a storage tank to obtain 1987.5 kl of a solution comprising 8.56 g/L of acetic acid as condensate water. In this manner, the condensate water discharged from the crystallization tubes and the concentration tubes was mixed at a predetermined ratio to prepare condensate water comprising 5 g/L to 15 g/L of acetic acid.

### Example 2. pH Adjustment of Condensate Water Comprising Acetic Acid

To 1 L of 5 g/L to 15 g/L of the acetic acid solution at 25°C prepared in Example 1, ammonia gas at the same temperature was introduced to adjust the pH.

For example, 4.3 g of ammonia gas was introduced into 1 L of 15 g/L of the acetic acid solution at 25°C to adjust the pH thereof to 5.

On the other hand, when the pH of the pH-adjusted condensate water comprising acetic acid prepared in Example 2 exceeds 6.0, equipment limitations occur in a reverse osmosis process, and thus the maximum pH of the condensate water that may be separated by the reverse osmosis process of Example 3 is 6.0.

### Example 3. Preparation of Retentate

The pH-adjusted condensate water comprising 5 g/L to 15 g/L of acetic acid, prepared in Example 2, was concentrated by a reverse osmosis process to obtain a retentate comprising 25 g/L to 45 g/L of acetic acid.

In detail, the equipment (Alfa Laval, RO 99 Type) used in the reverse osmosis process consisted of two membranes in series, each having a membrane area of 811 m² and 1217 m², and a pressure of 35 bar was applied. The pH-adjusted condensate water comprising 5 g/L to 15 g/L of acetic acid, prepared in Example 2, was fed into the reverse osmosis process equipment at 35°C and 60 kl/hr, and the maximum flow rate of a pump used at this time was 65 kl/hr.

### Example 4. Recovery of Acetic Acid Using Fermentation Broth or Fermentation Waste Liquor Comprising Acetic Acid

The retentate prepared in Example 3 was re-concentrated through evaporation to obtain a re-concentrated solution comprising 200 g/L to 400 g/L of acetic acid.

In detail, the concentration process through evaporation employed a method of using steam as a heat source to reach a temperature higher than the boiling point of the solution under the internal pressure conditions of a concentration tube (Hantec, multiple effect (6-effect) Falling Film Evaporator).

### Experimental Example 1. Recovery of Acetic Acid and Ammonia according to Acetic Acid Concentration and pH of Retentate

Recovery rates of acetic acid and ammonia according to the acetic acid concentration and pH of the retentate prepared by the reverse osmosis process in Example 3 were measured.

The acetic acid recovery rate (%) of retentate is determined by a ratio of the recovered acetic acid (g) in the retentate after the reverse osmosis process of Example 3 to the initial acetic acid (g) of the initial condensate water of Example 1.

The results are shown in Table 1 below.

**[Table 1]**

| Acetic acid concentration of condensate water (g/L) | pH of condensate water | Addition amount of ammonia to 1 L of condensate water (g) | Acetic acid recovery rate of retentate (%) |
|---|---|---|---|
| 10.0 | 4.5 | 0.95 | 50.88 |
| 15.0 | 4.5 | 1.45 | 53.98 |
| 10.2 | 5.5 | 2.42 | 77.44 |
| 15.2 | 5.5 | 3.6 | 72.70 |
| 10.0 | 6.0 | 3.6 | 79.61 |
| 15.0 | 6.0 | 5.1 | 79.71 |
| 5.4 | 4.5 | 0.52 | 41.33 |
| 5.4 | 5.5 | 1.28 | 70.23 |
| 5.3 | 5.5 | 1.2 | 75.79 |

As a result, as shown in Table 1, it was confirmed that when the pH of the condensate water comprising acetic acid of Example 2 was 4.6 to 6.0 and the initial acetic acid concentration of the condensate water waste liquor of Example 1 was 5 g/L to 15 g/L, the retentate showed an excellent acetic acid recovery rate.

It was also confirmed that when the pH of the condensate water increases, the retentate showed a more excellent acetic acid recovery rate.

On the other hand, when the pH of the pH-adjusted condensate water comprising acetic acid prepared in Example 2 exceeds 6.0, equipment limitations occur in the reverse osmosis process, and therefore, the maximum pH of the condensate water that may be separated by the reverse osmosis process of Example 3 is 6.0.

### Experimental Example 2. Recovery of Acetic Acid Using Fermentation Broth or Fermentation Waste Liquor Comprising Acetic Acid

The acetic acid recovery rates were compared according to the concentration of the condensate water comprising acetic acid of Example 1, the adjusted pH of the condensate water comprising acetic acid of Example 2, the acetic acid concentration of the retentate of Example 3, and the acetic acid concentration of the re-concentrated solution of Example 4.

The final acetic acid recovery rate (%) is determined by a ratio of the recovered acetic acid (g) of the re-concentrated solution of Example 4 to the acetic acid (g) of the initial condensate water of Example 1.

The results are shown in Table 2 below.

**[Table 2]**

| No. | Acetic acid concentration of condensate water waste liquor (g/L) | pH of condensate water | Addition amount of ammonia to 1L of condensate water (g) | Acetic acid concentration of retentate (g/L) | Acetic acid concentration of Re-concentrated solution (g/L) | Final acetic acid recovery Rate (%) |
|---|---|---|---|---|---|---|
| 1 | 5 | 5 | 2.2 | 25 | 200 | 62.78 |
| 2 | 5 | 5 | 2.2 | 30 | 200 | 58.65 |
| 3 | 5 | 5 | 2.2 | 45 | 200 | 61.01 |
| 4 | 5 | 5 | 2.2 | 45 | 300 | 51.21 |
| 5 | 5 | 6 | 2.9 | 25 | 200 | 83.01 |
| 6 | 5 | 6 | 2.9 | 25 | 300 | 59.08 |
| 7 | 5 | 6 | 2.9 | 30 | 200 | 75.16 |
| 8 | 5 | 6 | 2.9 | 30 | 300 | 60.87 |
| 9 | 5 | 6 | 2.9 | 45 | 200 | 85.25 |
| 10 | 5 | 6 | 2.9 | 45 | 300 | 69.94 |
| 11 | 5 | 6 | 2.9 | 45 | 400 | 54.63 |
| 12 | 10 | 5 | 3 | 25 | 200 | 56.51 |
| 13 | 10 | 5 | 3 | 30 | 200 | 54.17 |
| 14 | 10 | 5 | 3 | 45 | 200 | 62.01 |
| 15 | 10 | 6 | 3.6 | 25 | 200 | 74.64 |
| 16 | 10 | 6 | 3.6 | 30 | 200 | 69.59 |
| 17 | 10 | 6 | 3.6 | 30 | 300 | 52.52 |
| 18 | 10 | 6 | 3.6 | 45 | 200 | 81.90 |
| 19 | 10 | 6 | 3.6 | 45 | 300 | 64.35 |
| 20 | 15 | 4.6 | 3.59 | 24 | 200 | 52.00 |
| 21 | 15 | 5 | 4.3 | 25 | 200 | 58.98 |
| 22 | 15 | 5 | 4.3 | 30 | 200 | 55.30 |
| 23 | 15 | 5 | 4.3 | 45 | 200 | 62.87 |
| 24 | 15 | 6 | 5.1 | 25 | 200 | 74.73 |
| 25 | 15 | 6 | 5.1 | 30 | 200 | 71.63 |
| 26 | 15 | 6 | 5.1 | 30 | 300 | 55.05 |
| 27 | 15 | 6 | 5.1 | 45 | 200 | 78.55 |
| 28 | 15 | 6 | 5.1 | 45 | 300 | 67.10 |
| 29 | 15 | 6 | 5.1 | 45 | 400 | 56.66 |

As shown in Table 2, for example, in the case of No. 23, specifically, an acetic acid solution at a concentration of 15 g/L, 35°C, and pH 5 was fed to the reverse osmosis process equipment, and the final acetic acid recovery rate after re-concentration through the reverse osmosis process was 62.87%.

Through this, it was confirmed that when the pH of the condensate water comprising acetic acid of Example 2 was 4.6 to 6.0 and the initial acetic acid concentration of the condensate water of Example 1 was 5 g/L to 15 g/L, the final recovery rate was 50% or more.

### Comparative Example 1. Recovery of Acetic Acid Using Fermentation Broth or Fermentation Waste Liquor Comprising Acetic Acid

With regard to the concentration of the condensate water comprising acetic acid of Example 1, the adjusted pH of the condensate water comprising acetic acid of Example 2, the acetic acid concentration of the retentate of Example 3, and the acetic acid concentration of the re-concentrated solution of Example 4, the acetic acid recovery rate was intended to be examined under conditions other than the optimal conditions confirmed in Experimental Example 2.

The final acetic acid recovery rate (%) is determined by a ratio of the recovered acetic acid (g) of the re-concentrated solution of Example 4 to the acetic acid (g) of the initial condensate water of Example 1.

The results are shown in Table 3 below and FIG. 1.

**[Table 3]**

| No. | Acetic acid concentration of condensate water waste liquor (g/L) | pH of condensate water | Addition amount of ammonia to 1 L of condensate water (g) | Acetic acid concentration of retentate (g/L) | Acetic acid concentration of re-concentrated solution (g/L) | Final acetic acid recovery rate (%) |
|---|---|---|---|---|---|---|
| 1 | 15 | 4.5 | 1.45 | 45 | 194.5 | 32.62 |
| 2 | | | | | 211.2 | 32.30 |
| 3 | | | | | 230.6 | 31.84 |
| 4 | | | | | 253.3 | 31.18 |
| 5 | | | | | 279.9 | 30.20 |
| 6 | | | | | 311.0 | 28.72 |
| 7 | | | | | 346.8 | 26.44 |
| 8 | | | | | 386.9 | 22.95 |
| 9 | | | | | 429.6 | 17.69 |
| 10 | | | | | 472.5 | 10.15 |

As shown in Table 3 and FIG. 1, it was confirmed that when the pH of the condensate water comprising acetic acid of Example 2 was less than 4.6, the final recovery rate was less than 50%, regardless of the degree of re-concentration.

On the other hand, when the pH of the pH-adjusted condensate water comprising acetic acid prepared in Example 2 exceeds 6.0, equipment limitations occur in the reverse osmosis process, and therefore, the maximum pH of the condensate water that may be separated by the reverse osmosis process of Example 3 is 6.0.

In other words, it was confirmed that the final recovery rate was 50% or more only under the optimal conditions where the pH of the condensate water comprising acetic acid of Example 2 was 4.6 to 6.0 and the initial acetic acid concentration of the condensate water of Example 1 was 5 g/L to 15 g/L.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within the metes and bounds of the claims, or equivalents of such metes and bounds are therefore intended to be embraced by the claims.

## Claims

1. A method of recovering acetic acid from a fermentation broth or fermentation waste liquor, the method comprising the steps of:
(a) adding a basic substance to the fermentation broth or fermentation waste liquor comprising acetic acid to adjust the pH to 4.6 to 6.0; and
(b) concentrating the pH-adjusted fermentation broth or fermentation waste liquor by a reverse osmosis process to obtain an acetic acid solution.

2. A method of reusing acetic acid from a fermentation broth or fermentation waste liquor, the method comprising the steps of:
(a) adding a basic substance to a fermentation broth or fermentation waste liquor comprising acetic acid to adjust the pH to 4.6 to 6.0;
(b) concentrating the pH-adjusted fermentation broth or fermentation waste liquor by a reverse osmosis process to obtain an acetic acid solution; and
(c) introducing the acetic acid solution to a medium.

3. The method of claim 1 or 2, wherein the fermentation broth comprises an amino acid or a derivative thereof prepared by adding (i) O-acylhomoserine; and (ii) O-acylhomoserine converting enzyme or a microorganism expressing the same.

4. The method of claim 1 or 2, further comprising, before the step (a), (i) a step of preparing a fermentation broth comprising L-homocysteine, L-methionine, or a derivative thereof by using microorganisms in a fermentation medium comprising O-acylhomoserine, and (ii) a step of obtaining the fermentation broth or fermentation waste liquor comprising acetic acid of the step (a) from the fermentation broth; or the fermentation waste liquor obtained by removing L-homocysteine, L-methionine, or the derivative thereof from the fermentation broth.

5. The method of claim 4, wherein the step (ii) further comprises a step of evaporating and cooling the fermentation broth; or the fermentation waste liquor obtained by removing L-homocysteine, L-methionine, or the derivative thereof from the fermentation broth to obtain a condensate water comprising acetic acid.

6. The method of claim 1 or 2, wherein the basic substance is ammonia or sodium hydroxide.

7. The method of claim 1, wherein the fermentation broth or fermentation waste liquor comprising acetic acid comprises acetic acid at a concentration of 5 g/L to 15 g/L.

8. The method of claim 1 or 2, wherein the acetic acid solution obtained in the step (b) comprises acetic acid at a concentration of 25 g/L to 45 g/L.

9. The method of claim 1 or 2, wherein the step (b) comprises a step of re-concentrating the obtained acetic acid solution.

10. The method of claim 9, wherein the re-concentrated solution prepared in the re-concentration step comprises acetic acid at a concentration of 200 g/L to 400 g/L.
